# EUROPEAN PATENT SPECIFICATION

(11) **EP 2 089 101 B1**
(45) Date of publication and mention of the grant of the patent: **16.04.2014**
(21) Application number: 07749065.4
(22) Date of filing: 23.01.2007
(51) Int. Cl.: A61N 1/08

(54) **CONTROLLER AND SYSTEM FOR OBTAINING PRESCRIPTIVE ANALYSIS OF FUNCTIONALITY OF IMPLANTABLE MEDICAL DEVICE LEADS**
REGLER UND SYSTEM ZUR GEWINNUNG EINER VORGESCHRIEBENEN ANALYSE DER FUNKTIONALITÄT VON IMPLANTIERBAREN LEITUNGEN VON MEDIZINPRODUKTEN
APPAREIL DE CONTRÔLE ET SYSTÈME PERMETTANT D'OBTENIR UNE ANALYSE PRESCRIPTIVE DE LA FONCTIONNALITÉ DES CONDUCTEURS D'UN DISPOSITIF MÉDICAL IMPLANTABLE

(30) Priority: 31.10.2006 US 590741
(43) Date of publication of application: 19.08.2009
(73) Proprietor: Medtronic, Inc., Minneapolis, MN 55432-5604 (US)
(72) Inventor: GOETZ, Steven, M., North Oaks, MN 55127 (US); SMITH, Todd, V., Shoreview, MN 55126 (US); TORGERSON, Nathan, A., Andover, MN 55304 (US); BALL, Warren, W., Coon Rapids, MN 55448 (US)
(74) Representative: Hughes, Andrea Michelle
(86) International application number: PCT/US2007/001727
(87) International publication number: WO 2008/054438

(56) References cited:
- US-A- 5 003 975
- US-A- 5 507 786
- US-A- 5 755 742
- US-A1- 2006 116 733
- US-B1- 6 516 227
- US-B2- 6 721 600

## Description

### FIELD

The present invention relates generally to controllers and systems for implantable medical devices and, more particularly, to such controllers and systems for implantable medical devices having therapeutic electrodes.

### BACKGROUND

The medical device industry produces a wide variety of electronic devices for treating patient medical conditions. Depending upon medical condition, medical devices can be surgically implanted or connected externally to the patient receiving treatment. Medical professionals or other clinicians use medical devices alone or in combination with drug therapies and surgery to treat patient medical conditions. For some medical conditions medical devices provide the best, and sometimes the only, therapy to restore an individual to a more healthful condition and a fuller life. Examples of implantable medical devices designed to deliver therapeutic electrical stimulation include neurological stimulators, pacemakers and defibrillators.

Implantable medical devices configured to deliver therapeutic electrical stimulation commonly deliver therapy via electrodes positioned on one or more leads operatively connected to the implantable medical device. In some instances, the housing of the implantable medical device may also serve as an electrode or an electrode may be positioned on the housing. The electrodes or electrodes are commonly positioned in the patient's body during the same surgical procedure in which the implantable medical device is implanted.

The positioning of electrodes, and associated leads, is often an inexact procedure and may commonly be dependent on the particular, physiologic characteristics of the patient. In addition, electrodes may commonly be positioned within the patient without the medical professional or user conducting the procedure being capable of actually seeing where the electrodes are positioned. Instead, external aides such as fluoroscopes and/or endoscopes may commonly be employed to inform the medical professional or other user as to an approximate location of the electrodes.

Due to the inherent uncertainty involved in the placement of electrodes for an implantable medical device, implantable medical devices and the external controllers that interface with the devices are commonly operable to perform a test on the leads and electrodes to verify that the leads and electrodes are functioning properly and are positioned correctly. A common test is to check the impedance between pairs of electrodes During testing, an electrode can be driven with a signal having known electrical characteristics The signal may be measured, e g., on another electrode, and the impedance computed between electrodes using known fundamental relationships. The measured impedance value can give a medical professional or other user information relating to whether the electrodes involved in the test are positioned correctly and operating properly.

An external controller, or physician programmer, is commonly utilized in lead impedance tests Physician programmers can be similar in size and composition to a large laptop computer. The physician programmer provides a user interface via a display screen, and is manipulated by a medical professional via a vanety of inputs, such as buttons and touchscreens. The physician programmer commonly communicates with the implantable medical device via inductive telemetry. In order to accomplish this, a coil, operatively coupled to the controller, typically by a wire, is placed over a coil operatively coupled to the electronics in the implantable medical device, thereby establishing an inductive link over which data may be passed in either direction. Because physician programmers are typically not sterilized, the physician programmer itself is placed outside of the sterile field, only the coil and its housing is taken inside the sterile field, e.g., using a sterile bag.

For example, United States Patent Application Publication No. 2006/0036186, Goetz et al, Automatic Impedance Measurement of an Implantable Medical Device, discloses a method and controller for automating impedance measurements An entry for each electrode pair is displayed on a user interface Each electrode pair entry includes an identification of electrodes for an electrode pair, an associated value of impedance, and a value of current that is measured between the electrodes of a pair.

In another example, United States Patent No 5,891,179, Er et al, Method and Apparatus for Monitoring and Displaying Lead Impedance in Real-Time for an Implantable Medical Device, discloses a method and controller for displaying real-time graphical representations of variable lead impedance. Impedance values are calculated using Ohm's law or other related equations. Then the calculated impedance values are output to a graphic display for presentation thereby in graphical form or are output to a graphic printer, or both.

In another example, United States Patent Application Publication No 2003/0114899, Samuelsson et al, Programming System for Medical Devices, discloses a method and controller for displaying graphical representations of a quantity influenced by the operation of a medical device. Such quantities may include information derived from tests and diagnostics, such as an electrode impedance test.

In another example, United States Patent Application Publication No. 2005/0033385, Peterson et al, Implantable Medical Device Programming Apparatus Having a Graphical User Interface, discloses graphical displays of the operation of a medical device, such as a test of a device lead Results are organized according to the anatomical position of the lead, i.e, whether the lead is an atrial or ventricular lead, allowing the clinician to efficiently assess the functionally of all lead data by virtue of its grouping into precise anatomical categories.

In another example, United States Patent No 6,721,600, Jorgenson et al, Implantable Lead Functional Status Monitor and Method, discloses a system for obtaining trend data on the status of leads of an implantable medical device. The lead status measurement derives its data from various sources including lead impedance, non-physiologic sensed events, percentage of time the device is in mode switch, the results of capture management operation, sensed events, reversion paced counts, and refractory sense counts. The lead status measurement employs a set of weighted sum rules used by algorithms to process data from all of the above-mentioned sources to arrive at easily interpreted messages accessible to clinicians via an external programmer. Data from these sources identify lead conductor/connector interface issues and electrode/tissue interface issues indicative of lead-related mechanisms suggestive of impending or actual lead failure. The weights are "interpreted" for the user in the following manner either by indicating (1) lead-related parameters are all within range or operating normally; (2) one or more of the lead parameters are out-of-range and, thus, leads should be investigated, or (3) a number of lead parameters are out-of-range and a safety problem exists

Messages to the user refer to three types of lead-related conditions: either lead/conductor/connector messages, lead insulation messages or biological interface messages. Examples of such messages include: (1) high impedance (> 4000 ohms, 2x increase over reference, among others); (2) increase in threshold(s) above preset or programmed limit; and (3) reduction in R-wave and P-wave amplitude below preset or programmed limits. A controller according to the first part of claim 1 is known from US 2006/0116733 A1.

Summary information from a variety of trend data is therefore presented for the use of a medical professional.

### SUMMARY OF THE INVENTION

The present invention conducts inter-electrode impedance measurements in ways fundamentally similar to the inventions disclosed above. Once the impedance measurement is completed, however, in addition to the raw data, a prescriptive analysis, possibly including possible causes of any discrepancies in the measured data from preferred and anticipated results, is displayed on the external controller. Those prescriptive analyses go beyond identifying that a problem exists, instead adding interpretation of the results. The prescriptive analysis according to the invention includes determining a location of a short circuit of a lead relative to said implantable medical device, based on said impedance value and may suggest electrode combinations useful for effective therapy given that certain electrodes are not functional.

Like much of the above documents, the controller provides a range of impedance values considered normal, bounded on either end by values fixed for the test. In addition, a medical professional may specify the range of impedance values considered normal. Inter-electrode impedance tests may also be conducted at voltage and current levels that are used by the device to deliver therapy in order to determine functionality at operation voltage levels. The controller may then provide recommendations specific to the therapy to be delivered

The present invention provides a controller for an implantable medical device having a plurality of electrodes, the implantable medical device capable of delivering therapeutic stimulation to a patient, comprising a control module, a user interface operatively coupled to the control module, the user interface providing control of the control module by a medical professional or other user, and an electrode interface operatively coupled between the plurality of electrodes and the control module The control module uses the electrode interface to obtain a plurality of measurements of impedance values for a plurality of selected pairs of individual ones of the plurality of electrodes. The control module determines a prescriptive analysis using the plurality of measurements of impedance values of the selected pairs of individual ones of the plurality of electrodes comparative to a, preferably predetermined, range, and the user interface displays the prescriptive analysis

The prescriptive analysis identifies a possible cause of at least one of the plurality measurements of impedance values of the selected pairs of individual ones of the plurality of electrodes being outside of the range

The prescriptive analysis comprises a recommended action to at least in part rectify the at least one of the plurality of measurements of impedance values that is outside of the range.

The user interface displays the recommended action.

In an embodiment, the control module inhibits delivery of the therapeutic stimulation on at least one of the plurality of electrodes for which the prescriptive analysis determines is a cause of at least one of the plurality of measurements of impedance values of the selected pairs of individual ones of the plurality of electrodes being outside of the range.

In an embodiment, the control module inhibits delivery of the therapeutic stimulation on at least one of the plurality of electrodes for which one of the plurality of measurements of impedance values corresponding to the at least one pair of the selected pair of individual ones of the plurality of electrodes is outside of the range.

In an embodiment, the control module, under direction of the medical professional or other user via the user interface, inhibits at least one of the plurality of electrodes

In an embodiment, the user interface displays the plurality of measurements of impedance values of the plurality of selected pairs of individual ones of the plurality of electrodes.

There is also disclosed a method for delivering therapeutic stimulation to a patient using an implantable medical device having a plurality of electrodes, comprising the steps of obtaining a plurality of measurements of impedance values for a plurality of selected pairs of individual ones of the plurality of electrodes, determining a prescriptive analysis using the plurality of measured impedance values of the selected pairs of individual ones of the plurality of electrodes comparative to a, preferably predetermined, range, and communicating the prescriptive analysis to a user

There is a step of identifying a possible cause of at least one of the plurality of measured impedance values of the selected pairs of individual ones of the plurality of electrodes being outside of the range.

The prescriptive analysis comprises a recommended action to at least in part rectify the at least one of the plurality of measured impedance values that is outside of the range.

There is a step of displaying the recommended action.

There is a step of inhibiting delivery of the therapeutic stimulation on at least one of the selected pairs of individual ones of the plurality of electrodes for which the prescriptive analysis determines is a cause of at least one of the plurality of measured impedance values of the selected pairs of individual ones of the plurality of electrodes being outside of the range.

The inhibiting step inhibits delivery of the therapeutic stimulation, on at least one of the selected pairs of individual ones of the plurality of electrodes for which one of the plurality of impedance values corresponding to the at least one pair of the selected pair of individual ones of the plurality of electrodes is outside of the range

There is a step of preventing, under direction of the medical professional or other user via the user interface, at least one of the plurality of measurements of a selected pair of individual ones of the plurality of electrodes.

There is a step displaying the measured impedance values of the plurality of selected pairs of individual ones of the plurality of electrodes.

### BRIEF DESCRIPTION OF THE DRAWINGS

**Figure 1** shows an example of an implantable neurological stimulator implanted in the side of a patient, with electrodes positioned along the patient's spinal cord;
**Figure 2** shows an implantable neurological stimulator with a lead and lead extender and electrodes;
**Figure 3** shows a screen shot of a window for controlling an electrode impedance test of an implantable neurological stimulator;
**Figure 4** shows a screen shot of a window for displaying out-of-range results of an electrode impedance test of an implantable neurological stimulator;
**Figure 5** shows a screen shot of a window for displaying results of an electrode impedance test of an implantable neurological stimulator;
**Figure 6** shows a screen shot of a window for displaying recommendations for addressing failures that resulted during an electrode impedance test for an implantable neurological stimulator,
**Figure 7** shows a block diagram of a controller for an implantable medical device,
**Figure 8** shows a cutaway diagram of a lead with electrodes, and a lead extender, for an implantable medical device;
**Figure 9** is a flow chart for conducting a therapy impedance measurement for an implantable medical device; and
**Figure 10** is a flow chart for conducting an electrode impedance test for an implantable medical device.

### DETAILED DESCRIPTION

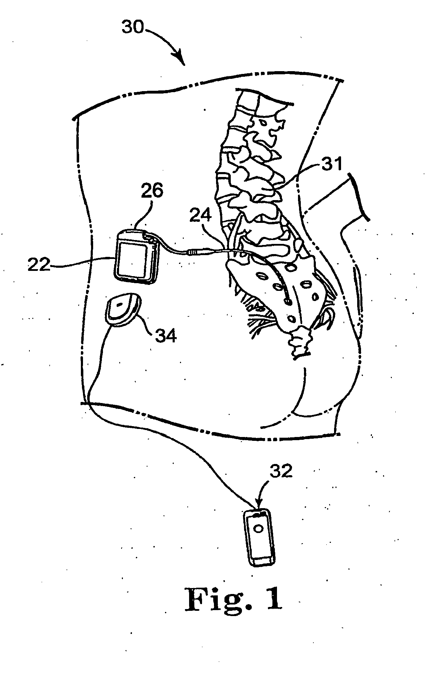

**Figure 1** shows the general environment of one rechargeable implantable medical device 20 embodiment. Implantable neurological stimulator 22 is shown, but other embodiments such as pacemakers and defibrillators and the like are also applicable. Implantable neurological stimulator 22 is implanted subcutaneously in side 28 of patient 30. Lead 24 is operatively coupled to implantable neurological stimulator 22 at header 26 Lead 24 is positioned along spinal chord 31 of patient 30 Controller 32, which may be a physician programmer or patient programmer, may become transcutaneously coupled to implantable neurological stimulator 22 via an inductive communication link through the tissue of patient 30 when controller 32 is placed in proximity to implantable neurological stimulator 22.

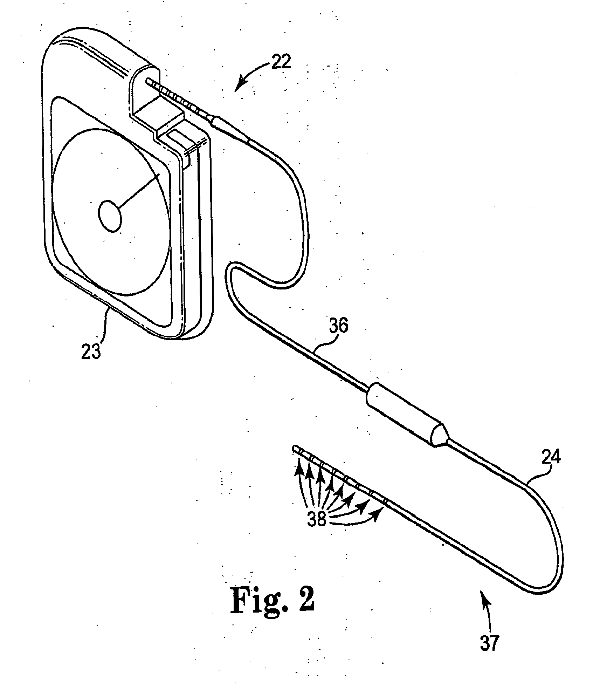

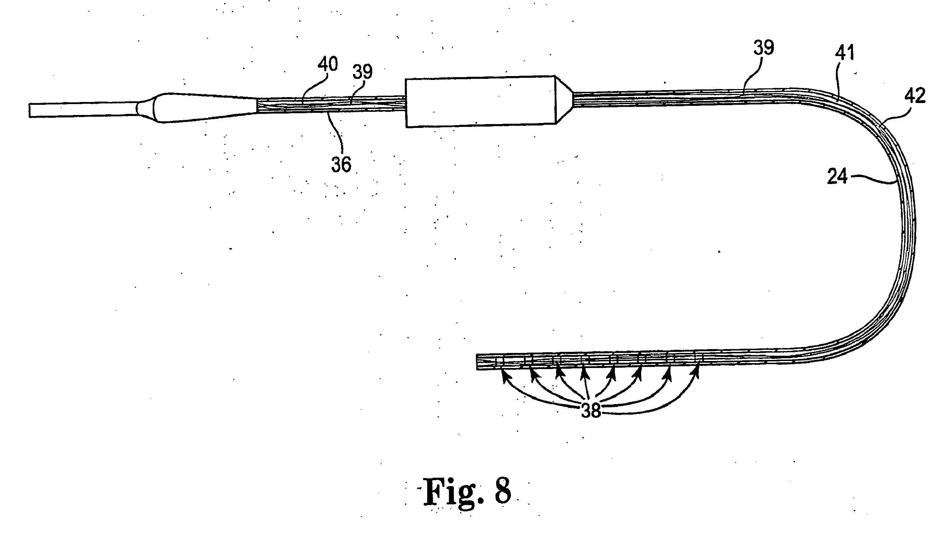

**Figures 2** and **8** show a closer view of implantable neurological stimulator 22 and lead 24, operatively coupled by extender 36. Electrodes 38 are mounted on distal end 37 of lead 24. Electrodes 38 are comprised of a conductive material, in an embodiment, metal, that come into direct contact with tissue of patient 30. Electrodes 38 are operatively coupled with implantable neurological stimulator 22 via header 26 through wires 39 comprised of conductive material that pass through the interior 41 of lead 24 and are operatively coupled with conductive wires 39 in the interior 40 of extender 36.

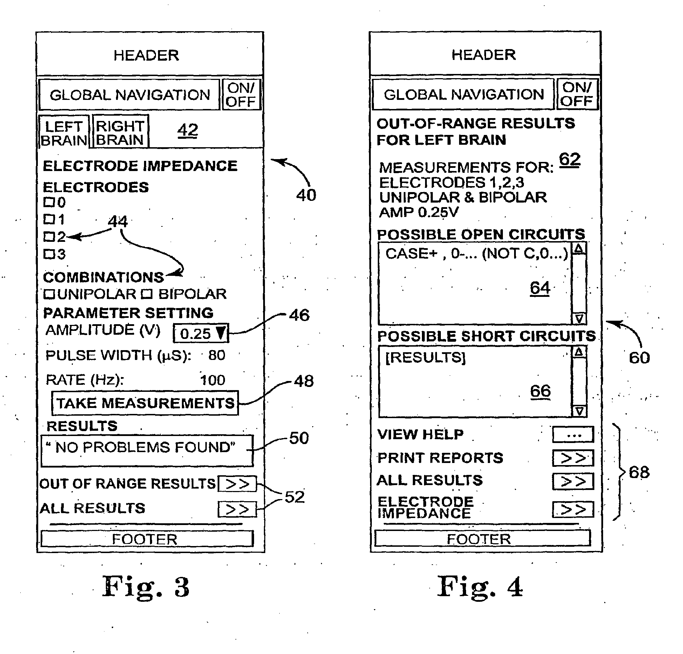

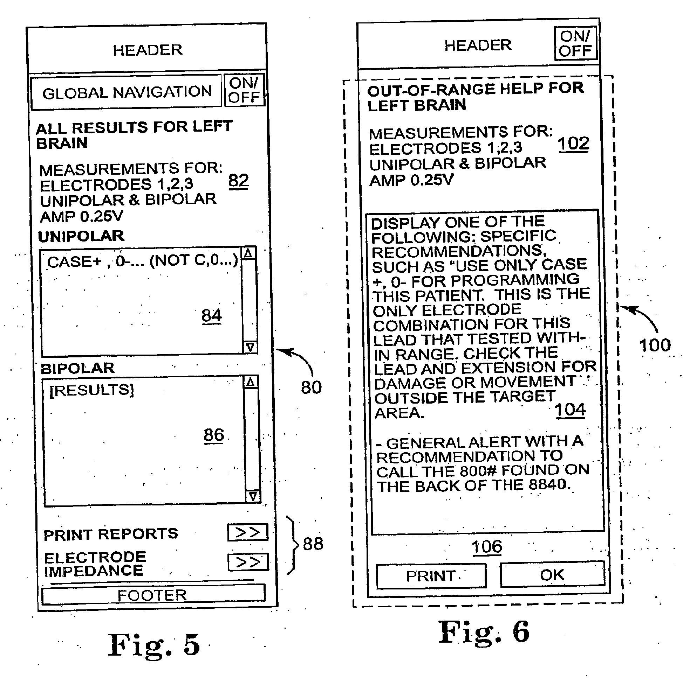

**Figure 3** shows electrode impedance panel 40 for neurological stimulator 22, in this case a deep brain stimulator. Pick menus 42 allow selection of different leads 24 to test. Pick boxes 44 allow the medical professional or other user to select which electrodes 38 will be tested and whether those electrodes 38 will be tested m unipolar or bipolar configuration Scroll menu 46 allows the medical professional or other user to set the voltage amplitude, and optionally, pulse width and frequency, at which the test will be conducted. Pressing button 48 begins the test according to the parameters that have been chosen on panel 40 After the test has completed a summary of the results is displayed in window 50, while buttons 52 give the medical professional or other user access to panel 60 (Figure 4) that displays all results that were out of the predetermined range and to panel 80 (Figure 5) that displays all results

In a typical electrode impedance test, each electrode 38 will be tested both in unipolar mode and bipolar mode Each electrode 38 in unipolar mode is paired up with neurological stimulator case 23 and the impedance between each electrode 38 and implantable neurological stimulator case 23 is measured and stored In addition, each electrode 38 an bipolar mode is paired up with every other electrode 38 and the impedance between each electrode 38-and every other electrode 38 is measured and stored. An exception may be that electrodes 38 that are located in different physiologic regions of the body, e.g., the head, the chest, are never paired and tested.

Figure 4 shows out of range results panel 60 for displaying the results of testing initiated from electrode impedance panel 40. Text 62 at top of out of range results panel 60 informs the medical professional or other user what test the current results pertain to by displaying which electrodes 38 were tested, in which mode electrodes 38 were tested and at which voltage amplitude electrodes 38 were tested. Possible open circuits window 64 lists possible locations, e.g., all possible locations, of open circuits that cause faults of tested electrodes 38. Possible short circuits window 66 lists possible locations, e.g., all possible locations, of short circuits that cause faults. Buttons 68 provide access to out of range help panel 100 (Figure 6), all results panel 80 (Figure 5) and electrode impedance panel 40, as well as a print command to print the data displayed on out of range results panel 60. ,

Open circuits are typically detectable when all measured impedance values for one electrode 38 are higher than the allowable maximum value. For instance, if all impedance values involving electrode (38) number, two exceed the maximum value and all impedance values not involving electrode 38 number two are within the allowable value, the controller could conclude that an open circuit existed on the path along which electrode (38) number two was operatively coupled with implantable neurological stimulator 22. Similarly, if all measured impedance values pertaining to electrodes. (38) number two and six exceeded the maximum value and all impedance values not involving electrodes (38) number two and six are within the allowable a values, the controller could conclude that both electrodes (38) number two and six were open.

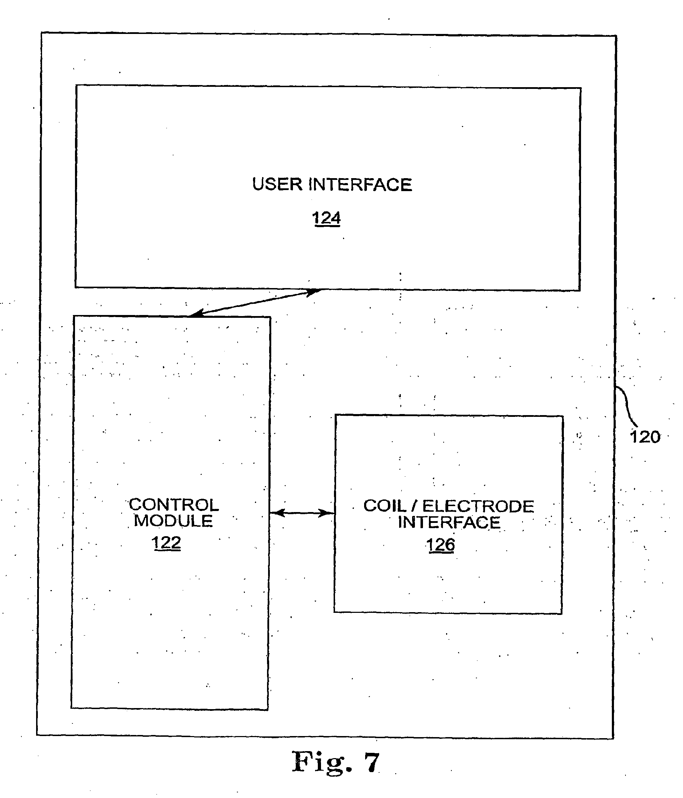

By contrast, short circuits are typically detectable when all measured impedance values involving those two electrodes (38) are lower than average and the measured impedance valve between the two electrodes 38 is below the minimum allowable value. For instance, if the average impedance between electrodes (38) is five hundred ohms, but between electrodes (38) four and five, and four and six, in bipolar, mode, and electrodes (38) five and case 23 and six and case 23 were all four hundred ohms, and the impedance between electrodes 38 five and six was below the allowable minimum value, controller 120 (Figure 7) could conclude that there is a short circuit between electrodes (38) five and six. Such short circuits can occur, among other reasons, because the electrodes 38 in question are physically touching, or insulation 42 between wires 39 operatively coupling electrodes 38 with implantable neurological stimulator 22 have frayed.

Occasionally, the results of testing may provide ambiguous results. For instance, if the impedance between electrodes (38) zero and two, three and two and between electrode (38) two and case 23 are all greater than the maximum allowable value, but the impedance between electrodes (38) one and two is within the allowable range, then it might not be clear what is the underlying cause of the issue. Controller 120 could return a message indicating that out of range values had been detected, but could be unable to offer definitive prescriptive guidance.

Figure 5 shows all results panel 80, for displaying all results of testing initiated from electrode impedance panel 40, regardless of whether testing resulted in an indication of failure or failures or not. Text 82 at the top of all results panel 80 informs the medical professional or other user to what test the current results pertain by displaying which electrodes 38 were tested, in which mode electrodes 38 were tested and at which voltage amplitude electrodes 38 were tested. Results are displayed in one of two windows 84, 86 depending on if the test mode was unipolar 84 or bipolar 86. Buttons 88 provide access to electrode impedance panel 40 and a print command.

Figure 6 shows out of range help panel 100 for displaying recommendations for addressing the causes of any failures that derive from tests initiated from electrode impedance panel 40. Text 102 at the top of out of range help panel 100 informs the medical professional or other user to what test the current results pertain by displaying which electrodes 38 were tested, in which mode electrodes 38 were tested and at which voltage amplitude electrodes 38 were tested. Window 104 shows recommended courses of action based on the results of the testing. Buttons 106 give access to a print command, and OK to return to out of range results panel 60.

Out of range help panel 100 may display analyses in conversational language dependent on the results of testing. For instance, if no faults are found, the result might read "No Problems Found." If an open circuit was found on electrode 38 number two then the result might read "Combinations containing electrode 2 have measured with high impedance High impedances are often the result of a broken lead wire or an issue with the connector." If all electrodes 38 on a particular lead 24 register with high impedance, the result might read "All electrodes on the right lead measure high impedance. There may be an issue with the lead or extension connector. This could be caused by a lead or extension only partially inserted into a connector."

Where the results indicate electrodes 38 number five and six are shorted, the result might read "The impedance between electrodes 5 and 6 appears to be unusually low. This may result from a short or insulation issue on those two electrodes. This may result from leads touching or crossing in the tissue." If the results are ambiguous as to the cause, the result might be "Out of range values have been detected" without further elaboration.

Where a clear conclusion can be drawn from the data, recommendations may be made about therapeutic settings. For instance, if electrode (38) number two appeared to be open, the recommendation may include "Attempting to deliver therapy on electrode 2 may provide unexpected or inconsistent results. Do you wish to disable electrode 2 on the programming screen? Do you wish to review Left Lead settings now?"

Further, depending on the information available, controller 120 may be able to determine the location of a lead short or open circuit. If a short occurs in close proximity to implantable neurological stimulator 22, the measured impedance value will tend to be very small. If a short occurs relatively far away from implantable neurological stimulator 22, then loop resistance will tend to be high. Based on these relationships, the analysis of the fault may be modified to identify the location of the fault.

**Figure 7** shows a block diagram of the functional blocks of controller 120. Control module 122 comprises a variety of off the shelf electronic components commonly found in a variety of commercial applications, such as personal computers. These electronic components include: a microprocessor, RAM, ROM and hard disks. These off the shelf components are integrated into control module 122 and additional operational features are added via custom electronics. These custom electronics are comprised of off the shelf integrated circuits and discrete components, and programmable components, such as FPGAs and DSPs, and custom integrated circuits and PCBs.

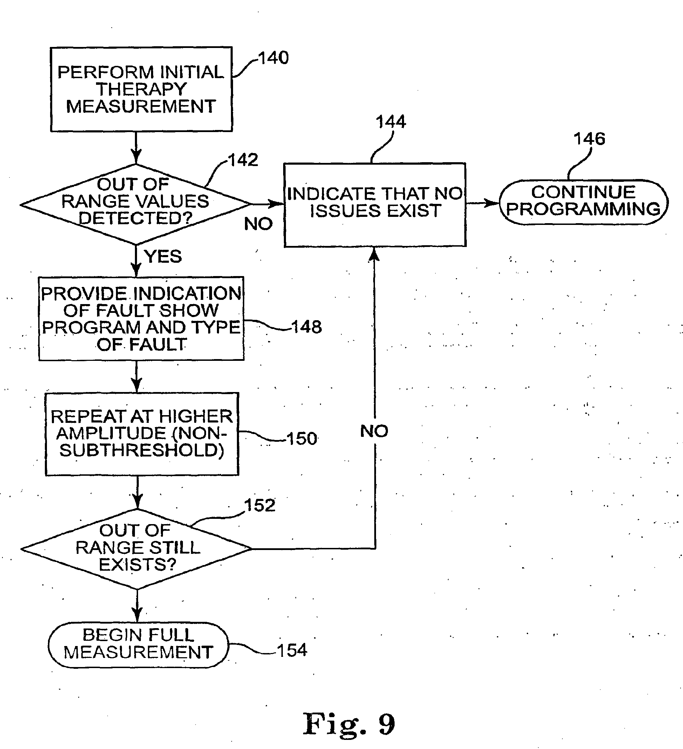

**Figure 9** is a flow chart for measuring therapeutic impedance or the impedance electrodes 38 see when delivering stimulation of the same sort as when implantable neurological stimulator 22 is delivering therapy to the patient. It is often desirable to verify that the electrodes 38 will deliver therapy as anticipated. Sometimes lipids form on electrodes 38 and while voltage levels below a threshold value will not penetrate the lipid layer, thereby suggesting high impedance, voltage levels higher than the threshold value will pass through, thereby suggesting acceptable impedance. The allowable range of impedance values will vary depending on the specific characteristics of the therapy to be delivered.

Initially, implantable neurological stimulator 22 performs a partial therapy measurement (140) involving minimal testing that still tests all electrodes 38 at a voltage that is below the allowable minimum value for the therapy that is being tested. If all of the resulting measured impedance values are within the allowable range (142) controller 120 indicates that no issue exists (144) and the user may continue programming implantable neurological stimulator 22 (146).

If any result is out of range, however, controller 120 indicates the fault (148) and the test is repeated at an amplitude that is at or above the minimum threshold value (150). If all results now pass (152) then controller 120 indicates that no issue exists (144) and the user may continue programming implantable neurological stimulator 22 (146) However, if any results fail (154) then a full electrode impedance test is conducted, as described above, at voltage and current levels that are commonly used in the prescribed therapy. Optionally, iterative testing may be done at higher voltage and/or current levels and potentially multiple, for example three, levels.

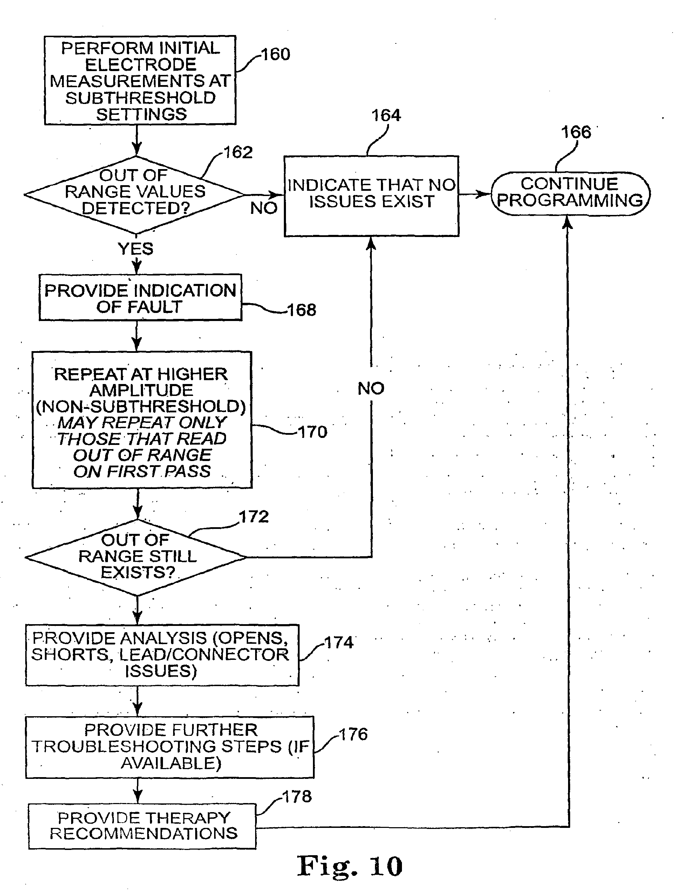

**Figure 10** is a flow chart for conducting a standard electrode impedance test Initially, implantable neurological stimulator 22 performs a partial impedance measurement (160) involving minimal testing that still tests all electrode 38 pairs at a voltage that is below the allowable minimum value. If all of the resulting measured impedance values are within the allowable range (162) controller 120 indicates that no issue exists (164), the user may continue programming implantable neurological stimulator 22 (166).

If any result is out of range, however, controller 120 indicates (168) the fault and the test is repeated at an amplitude that is at or above the minimum threshold value (170). If all results now pass (172), then controller 120 indicates that no issue exists (164) and the user may continue programming (166) implantable neurological stimulator 22. If not, the user may specify that the impedance test may be repeated to verify the fault. If the results are still out of range after a repeat of the test, or if a repeat is not allowed, then controller 120 provides (174) an analysis of the faults specifying, where possible, whether the results are likely due to reasons described above. Controller 120 then provides troubleshooting advice (176), of the types described above, and provides therapy recommendations (178) of the types described above. The user may then proceed to act on the provided analysis and advice (166).

While most implantable neurological stimulators 22 conduct impedance tests as a function of voltage, some implantable neurological stimulators 22 are capable of conducting impedance tests as a function of current. Accordingly, in an alternative mode, electrode impedance testing may be performed as a function of current. This function, however, may only be utilized when testing an implantable neurological stimulator 22 operable to test electrode impedance as a function of current.

Further, while the results of electrode impedance tests are commonly compared against a fixed allowable range, an adaptive algorithm that compares all measured impedance values against the average of the measured impedance values. Results that vary from the average may be flagged as suspect, deserving of further analysis.

## Claims

1. A controller for an implantable medical device (22) capable of delivering a therapeutic output to a patient, said implantable medical device having a lead (24) having a plurality of electrodes (38), said controller having:
a control module (122), operatively coupled to said implantable medical device, being programmable by a medical professional to specify, at least in part, said therapeutic output to be delivered to said patient;
a user interface (124) operatively coupled to said control module, said user interface providing control of said control module by a medical professional or other user;
an electrode interface (126) operatively coupled between said plurality of electrodes and said control module;
said control module being selectable by said medical professional to perform one of a plurality of procedures;
said control module determining impedance values of a selected set of individual ones of said plurality of electrodes determining a prescriptive analysis using said impedance value of said selected set of individual ones of said plurality of electrodes comparative to a range;
said user interface displaying said prescriptive analysis and
said control module presenting said interface with said at least some of a plurality of tasks to be performed by said medical professional based upon a selected one of said plurality of procedures; **characterized in that** said prescriptive analysis includes determining a location of a short circuit of said lead relative to said implantable medical device, based on said impedance value.

2. A system capable of delivering a therapeutic output to a patient, having:
an implantable medical device capable of delivering said therapeutic output to said patient;
a controller as in claim 1.

3. A controller or system as in either of claims 1 or 2 wherein said interface only includes tasks to be performed by said medical professional that are associated with said selected one of said plurality of procedures.

4. A controller or system as in claim 3 wherein said interface presents said tasks in a chronological order of implementation by said medical professional.

5. A controller or system as in claim 4 wherein said interface hides tasks not associated with said selected one of said plurality of procedures.

6. A controller or system as in claim 5 wherein said interface also provides an option to said medical professional to select any of said plurality of tasks following selection of said selected one of said plurality of procedures.

7. A controller or system as in claim 6 wherein an otherwise hidden task selected under said option again is again hidden when said medical professional returns to said selected one of said plurality of procedures.

## Patentansprüche

1. Steuereinheit für eine implantierbare medizinische Vorrichtung (22), die einem Patienten eine therapeutische Ausgabe verabreichen kann, wobei die implantierbare medizinische Vorrichtung eine Leitung (24) mit mehreren Elektroden (38) besitzt, wobei die Steuereinheit besitzt:
ein Steuermodul (122), das betriebstechnisch an die implantierbare medizinische Vorrichtung gekoppelt ist, die durch einen medizinischen Fachmann programmierbar ist, um zumindest teilweise die an den Patienten zu verabreichende therapeutische Ausgabe zu spezifizieren;
eine Benutzeroberfläche (124), die betriebstechnisch an das Steuermodul gekoppelt ist, wobei die Benutzeroberfläche die Steuerung des Steuermoduls durch einen medizinischen Fachmann oder einen anderen Benutzer bereitstellt;
eine Elektrodenschnittstelle (126), die betriebstechnisch zwischen die mehreren Elektroden und das Steuermodul gekoppelt ist;
wobei das Steuermodul durch den medizinischen Fachmann auswählbar ist, einen von mehreren Vorgängen auszuführen;
wobei das Steuermodul Impedanzwerte einer ausgewählten Gruppe von einzelnen der mehreren Elektroden bestimmt, die eine verordnende bzw. präskriptive Analyse unter Verwendung der Impedanzwerte der ausgewählten Gruppe der einzelnen der mehreren Elektrode im Vergleich zu einem Bereich bestimmen;
wobei die Benutzeroberfläche die verordnende Analyse anzeigt und
das Steuermodul die Schnittstelle mit zumindest einigen von mehreren Aufgaben, die durch den medizinischen Fachmann aufgrund eines ausgewählten der mehreren Vorgänge auszuführen sind, darstellt;
**dadurch gekennzeichnet, dass**
die verordnende Analyse enthält, einen Ort eines Kurzschlusses der Leitung in Bezug auf die implantierbare medizinische Vorrichtung aufgrund des Impedanzwertes zu bestimmen.

2. System, das einem Patienten eine therapeutische Ausgabe verabreichen kann, mit:
einer implantierbaren medizinischen Vorrichtung, die dem Patienten die therapeutische Ausgabe verabreichen kann;
einer Steuereinheit nach Anspruch 1.

3. Steuereinheit oder System nach einem der Ansprüche 1 oder 2, wobei die Schnittstelle nur durch den medizinischen Fachmann auszuführende Aufgaben enthält, die einem der mehreren Vorgänge zugeordnet sind.

4. Steuereinheit oder System nach Anspruch 3, wobei die Schnittstelle die Aufgaben in einer chronologischen Reihenfolge des Umsetzens durch den medizinischen Fachmann darstellt.

5. Steuereinheit oder System nach Anspruch 4, wobei die Schnittstelle Aufgaben ausblendet, die nicht dem ausgewählten der mehreren Vorgänge zugeordnet sind.

6. Steuereinheit oder System nach Anspruch 5, wobei die Schnittstelle dem medizinischen Fachmann außerdem eine Option bereitstellt, eine beliebige der mehreren Aufgaben anschließend an die Auswahl des ausgewählten der mehreren Vorgänge auszuwählen.

7. Steuereinheit oder System nach Anspruch 6, wobei eine anderenfalls ausgeblendete Aufgabe, die unter der Option wieder ausgewählt wurde, wieder ausgeblendet wird, wenn der medizinische Fachmann zu dem ausgewählten der mehreren Vorgänge zurückkehrt.

## Revendications

1. Contrôleur pour un dispositif médical implantable (22) capable de délivrer une sortie thérapeutique à un patient, ledit dispositif médical implantable ayant une dérivation (24) comprenant une pluralité d'électrodes (38), ledit contrôleur comprenant :
un module de commande (122), couplé de manière opérationnelle audit dispositif médical implantable, étant programmable par un professionnel médical pour spécifier, au moins en partie, ladite sortie thérapeutique à délivrer audit patient ;
une interface utilisateur (124) couplée de manière opérationnelle audit module de commande, ladite interface utilisateur fournissant une commande dudit module de commande par un professionnel médical ou un autre utilisateur ;
une interface d'électrode (126) couplée de manière opérationnelle entre ladite pluralité d'électrodes et ledit module de commande ;
ledit module de commande étant sélectionnable par ledit professionnel médical pour exécuter une procédure parmi une pluralité de procédures ;
ledit module de commande déterminant des valeurs d'impédance d'un ensemble sélectionné d'électrodes individuelles parmi ladite pluralité d'électrodes déterminant une analyse prescriptive en utilisant ladite valeur d'impédance dudit ensemble sélectionné d'électrodes individuelles parmi ladite pluralité d'électrodes comparativement à une plage ;
ladite interface utilisateur affichant ladite analyse prescriptive ; et
ledit module de commande présentant ladite interface avec lesdites au moins certaines tâches parmi une pluralité de tâches exécutées par ledit professionnel médical sur la base d'une procédure sélectionnée parmi ladite pluralité de procédures ; **caractérisé en ce que** ladite analyse prescriptive comprend de déterminer un emplacement d'un court-circuit de ladite dérivation par rapport audit dispositif médical implantable, sur la base de ladite valeur d'impédance.

2. Système capable de délivrer une sortie thérapeutique à un patient, comprenant :
un dispositif médical implantable capable de délivrer ladite sortie thérapeutique au dit patient ;
un contrôleur comme dans 1 revendication 1.

3. Contrôleur ou système comme dans l'une ou l'autre des revendications 1 ou 2, dans lequel ladite interface comprend uniquement des tâches à exécuter par ledit professionnel médical qui sont associées à ladite procédure sélectionnée parmi ladite pluralité de procédures.

4. Contrôleur ou système comme dans la revendication 3, dans lequel ladite interface présente lesdites tâches dans un ordre chronologique de mise en oeuvre par ledit professionnel médical.

5. Contrôleur au système comme dans la revendication 4, dans lequel ladite interface masque des tâches n'étant pas associées à ladite procédure sélectionnée parmi ladite pluralité de procédures.

6. Contrôleur ou système comme dans la revendication 5, dans lequel ladite interface fournit également une option audit professionnel médical pour sélectionner l'une quelconque parmi ladite pluralité de tâches à la suite de la sélection de ladite procédure sélectionnée parmi ladite pluralité de procédures.

7. Contrôleur ou système comme dans la revendication 6 dans lequel une tâche autrement masquée sélectionnée à nouveau sous ladite option est à nouveau masquée lorsque ledit professionnel médical revient à ladite procédure sélectionnée parmi ladite pluralité de procédures.
